Europäisches Patentamt

(19) European Patent Office

Office européen des brevets

(11) Numéro de publication: **0 304 386**
**A2**

(12) **DEMANDE DE BREVET EUROPEEN**

(21) Numéro de dépôt: **88500048.9**

(22) Date de dépôt: **20.05.88**

(51) Int. Cl.⁴: **A 61 M 5/31**

(30) Priorité: **22.05.87 ES 8701507**
**09.12.87 ES 8703522**

(43) Date de publication de la demande:
**22.02.89 Bulletin 89/08**

(84) Etats contractants désignés:
**AT BE CH DE FR GB GR IT LI LU NL SE**

(71) Demandeur: **Rovira Mestres, Ramon**
**rue Juan XXIII n. 15-19**
**E-08950 Esplugues de Llobregat (Barcelona) (ES)**

THERMACROME DE ESPANA, S.A.
**rue Juan XXIII n. 15-19**
**08950 Esplugues de Llobregat (Barcelona) (ES)**

**Borras Colldefors, Ramon**
**rue Juan XXIII n. 15-19**
**08950 Esplugues de Llobregat (Barcelona) (ES)**

(72) Inventeur: **Rovira Mestres, Ramón**
**Juan XXIII ns. 15-19**
**E-08950 Esplugues de Llobregat Barcelona (ES)**

**Borras Colldefors, Ramôn**
**Juan XXIII ns. 15-19**
**E-08950 Esplugues de Llobregat Barcelona (ES)**

(74) Mandataire: **Manresa Val, Manuel**
**Gerona n. 34**
**E-08010 Barcelona (ES)**

(54) Seringue pour l'injection de fluides, comprenant un mécanisme la rendant non réutilisable.

(57) Seringue pour l'injection de fluides, à mécanisme appliqué à son inutilisation programmée.

Consiste en une seringue qui comporte une tige de manoeuvre 5, raccordé à un piston 4, avec un jeu entre les deux éléments, de façon telle que les mouvements en recul ou avancement de ladite tige 5, fournissent des déplacements relatives, à longueur délimitée de ladite tige 5, par rapport au piston 4, intégrant un élément auxiliaire 8, avec ses extrémités reliées aux piston 4 et tige 5, par un raccordement spécifique de friction ou engrenage, ledit élément auxiliaire 8, se déplaçant au long du corps de la seringue à conséquence du jeu entre tige et piston, avançant dans un sens unique, par étapes, en constituant l'ensemble tige, pis ton et élément auxiliaire, un mécanisme au moyen duquel il est possible d'agir sur divers activateurs pour inutiliser la seringue.

EP 0 304 386 A2

Bundesdruckerei Berlin

## Description

Ce brevet d'invention concerne une seringue pour l'injection de fluides, à mécanisme appliqué à son inutilisation programmée, consistant en une seringue forcément rejetable, son mécanisme restant inutilisé après avoir été employé, spécialement dessiné pour reduire le risque de transmision de maladies infectieuses.

Il faut remarquer, que la seringue proposée, a une structure générale, totalement équivalente à celles qui sont utilisées actuellement;elle sera présenté d'aprés les normes, dans un emballage stérile, et, étant, par ses característiques constitutives spéciales, obligatoirement pour un seul usage, elle garantit une utilisation, dans tous les cas, dans des conditions d'hygiène maximale.

Par ailleurs, cette seringue a un coût de fabrication similaire à celui des seringues conventionelles, en incorporant complémentairement seulement une ou deux pièces, selon les cas, simples d'obtenir.

Antécédents de seringues appliquées à la fin expliquée, qui aient été constatés, sont des seringues intègrant un ou plusieurs réservoirs, contenant des produits à appliquer, si bien lesdites seringues sont, par leur structure, coût et opératoire, tout à fait différentes de celle proposée par l'invention.Comme exemples desdites seringues, peuvent être cités les brevets USA n° 3.941.128 et espagnol n° 531.282.

On peut faire référence aussi aux brevets français n° 2.298.340 qui décrit une seringue à inutilisation volontaire, australien n° 16.859 et allemand n° 1.142.218.

La seringue objet de l'invention est formée par un corps tubulaire allongé, creux, terminé à une de ses extremités par un conduit adapte pour recevoir, branchée, une aiguille creuse (avantageusement integrée dans ledit conduit), ayant une extremitée en biais, et disposée en son intérieur, en montage coulissant, un piston, hermétiquement réglé, raccordé à une tige de manoeuvre.Il s'agit jusqu'à ce point d'une structure et pièces composantes tout à fait conventionelles, singularisée en ce qu'une portion à l'extremitée de ladite tige de manoeuvre, est mutuellement reliée au piston ou à un corps solidaire de celui-ci, par un raccordement fournissant un déplacement relatif, bidirectionnel, à longueur délimitée, de cette portion de l'extrémité de la tige, par rapport au piston ou corps solidaire avec lui, en correspondance avec toute la manoeuvre en recul ou avancement de la tige de manoeuvre.La seringue est caracterisée de plus en ce qu'elle intègre un élément auxiliaire, monté avec la possibilité de coulissement le long du corps de la seringue, associé par ses deux extrémités, à des raccordements spécifiques à friction ou engrenage, au piston et à l'éxtremité de la tige de manoeuvre, qui possède pour cela des moyens différenciés de retenue et de passage à travers lui, dudit elément auxiliaire, lequel à cause du jeu entre la tige et le piston, lorsque la seringue opère, est soumis à des efforts déterminant des déplacements linéaires correspondants, allant en un seul sens.A cet effet, cet élément auxiliaire adopte une configuration qui contribue à l'unidirectionalité et longueur de ses déplacements, pas à pas, le long du corps de la seringue.

L'ensemble formé par la tige de manoeuvre, piston et élément auxiliaire, constitue un mécanisme, au moyen duquel il est possible d'activer divers activateurs, pour inutiliser la seringue au bout d'un certain nombre de séquences préalablement établiés.Ainsi, par exemple, après un certain nombre de courses préalablement fixées par programme, dans les deux sens, consécutives, de la tige de manoeuvre, prope à l'opératoire conventionelle de la seringue, l'élément auxiliaire s'est déplacé jusqu'à un point en provoquant l'inhabilité de la fonction d'aspiration de la seringue, ou en éliminant le contrôle de la tige sur le piston.

Le corps de la seringue, intègre des moyens tels que des configurations d'enclenchement, pour empêcher un mouvement rélatif, accidentel, entre la tige de manoeuvre et le piston, jusqu'au moment d'utilisation de la seringue.Une configuration de l'embout ou d'une autre partie de la seringue est également prévue, qui empêche l'extraction du piston et/ou de la tige de manoeuvre à l'intérieur de son corps.

Ci-dessous, une description détaillée de la seringue proposée, est realisée à l'aide d'un jeu de dessin, où ont été dessinées des variantes de possibles réalisations qui doivent être entendues à simple titre d'exemple illustratif et non limitatif.Dans les figures de ces dessins on a représenté ce qui suit:

La figure 1 montre en section une seringue d'après cette invention.

La figure 2 illustre à une échelle plus grande, un détail de l'immobilisation de la tige de manoeuvre, par rapport à la paroi du corps tubulaire de la seringue jusqu'au moment de l'utilisation de ladite seringue.

Dans les figures 3 à 6, la seringue à laquelle nous nous rapportons, est dessiné a diverses stades opératoires, en ordre, jusqu'au moment ou se produit son inhabilité.

Les figures 7 et 8, montrent en coupe partielle, les deux pistons utilisés dans cette seringue.

La figure 9, illustre en coupe une variante de seringue, où la tige de manoeuvre reste reliée a un corps rigide lequel à son tour s'accouple au piston, en présentant également une configuration alternative de l'élément auxiliaire et des moyens pour le retenir.

Les figures 10 et 11 sont descriptives d'une variante de seringue où les déplacements de l'élément auxiliaire provoquera la séparation entre la tige de manoeuvre et le piston.

Les figures 12 et 13 montrent en coupe deux variantes de seringue caracterisées par la forme qu'adopte l'élément auxiliaire et les moyens de retenue de celui-ci a l'extremité de la tige de manoeuvre.

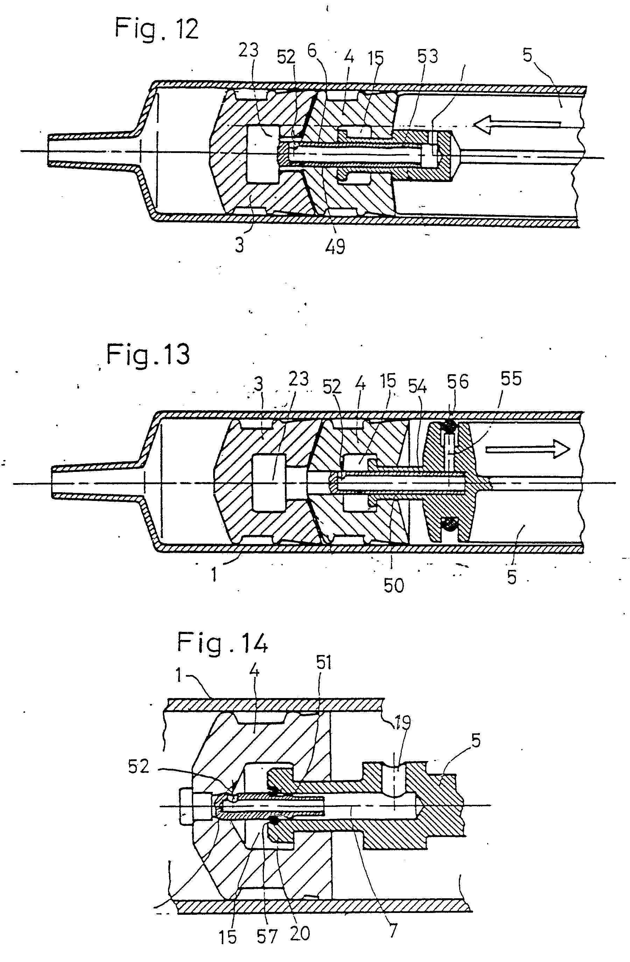

Finalement, dans la figure 14 un détail est dessiné d'une réalisation ultérieure caracterisée à nouveau par une configuration determinée de l'élément auxiliaire et moyens de raccordement avec l'extrémité de la tige de manoeuvre.

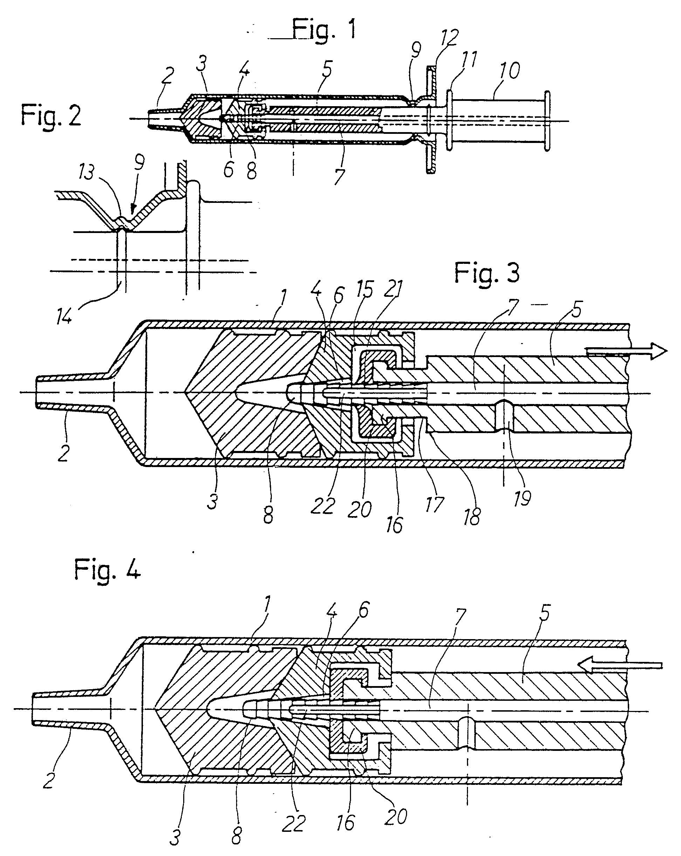

Conformément à l'exposé et en faisant référence à la figure 1, la seringue proposée comporte un corps tubulaire 1, terminé à une de ses extrémités par un conduit 2, adapté pour recevoir branchée une aiguille creuse, qui n'a pas été représentée, conventionnelle, qui comporte à l'intérieur de ce corps 1. un prémier piston 3, libre, et un deuxième piston 4, reliée à une tige de manoeuvre 5, ces deux éléments 4, 5 ayant des conduits 6, 7, alignés sur l'axe du corps 1, dans lequels restent introduits, avec raccord à friction, les extrémités d'un élément auxiliaire 8, monté avec possibilité de glissement en avançant dans un seul sens.On observe dans cette figure que après de l'embout du corps tubulaire 1, il présente un étranglement 9, annulaire, défini après l'installation a l'intérieur de ce corps 1, des éléments 4, 5 et 8 cités et executé par exemple par une opération de façonnage thermique, dans le cas où un matériau thermoplastique adéquat serait utilisé pour constituer 1.On peut voir par ailleurs dans la figure que la poignée 10, de la tige de manoeuvre 5, dispose d'une saillie annulaire 11, qui agit comme butée limitant l'avancement, en butant contre l'embout de la seringue, où aparaissent des rebords 12, d'après la configuration conventionnelle et pour que la seringue soit plus facile à saisir.

Dans la figure 2, on peut voir que sur le côté interne de la zone étranglée 9, du corps tubulaire 1, un rebaissement annulaire 13 est défini correspondant positionnellement avec une saillie 14, coaxiale à la tige de manoeuvre 5, qui constituent une configuration mâle-femelle d'enclenchement, qui empêche les déplacements accidentels entre lesdits tige 5, et piston 4, avant l'utilisation de la seringue, moment auquel lorsqu'on exerce une traction exprès sur la tige 5, avec sa poignée 10, ces moyens d'immobilisation sont désaccouplés, car on emploie des materiaux élastiques pour les deux.

Dans la figure 3 on apprécie avec détail qu'à l'intérieur du piston 4, une cavité 15 est définie, où est logée et retenue une extrémité grossie 16, de la tige 5.L'amplitude de la cavité 15, et la configuration de la portion du bout de la tige 5, qui après la portion grossie 16, présente un tronçon 17, ayant une coupe plus petite que le reste de cette tige 5, avec un raccordement par un échelon droit 18, déterminant qu'à chaque course en arrière ou en avant de cette tige 5, sa portion du bout grossie 16, se déplace d'une façon équivalente en arrière et en avant, à l'intérieur de la cavité 15, et par rapport au piston 4, une distance délimitée par la longueur du tronçon 17 et /ou l'amplitude de la cavité 15, avant que la tige 5, entraîne ou pousse ce piston 4.

On observe également sur cette figure 3, le conduit 7, axial à la tige 5, avec une sortie 19, à prise atmosphérique, et aussi le capuchon 20, en matériau élastique, accouplé recouvrant ladite portion grossie 16, qui parachève cette tige 5.Ce capuchon 20 présente un orifice de passage 21, qui reste en face du conduit 7, de la tige, et à travers celui-ci se trouve placé un elément auxiliaire tubulaire, qui présente sa face externe échelonnée selon une pluralité de secteurs tronconiques, égaux, raccordés en continuité, avec un sillon 22, générateur qui commence à un de ces bouts.L'autre bout de cet élément 8 reste introduit au sein du conduit 6, tronconique, parachevé en une portion cylindrique, (ou l'élément 8 ajuste avec fermeture hermetique), opérée à travers le piston 4, réalisé en un matériau plastique, avec conditions d'actuation differentes que celles du capuchon 20, cité.Etant donnée l'orientation des échelons tronconiques de l'élément auxiliaire 8, comme montre la figure 3, qui est descriptive de la première phase d'actionnement de la seringue, lorsqu'on tire en arrière la tige de manoeuvre 5, l'élément auxiliaire 8, reste fixe, par son raccordement à majeur friction, ajusté à la section cylindrique du bout du conduit 6, tandis que le capuchon 20 cède élastiquement et en même temps que le bout 16, de la tige 5 se déplace par rapport à ce piston et élément auxiliaire 8, en franchissant un échelon de ce dernier.

Un mouvement ultérieur de poussée de la tige de ma noeuvre engendrera un avancement de l'élémemt auxiliaire 8, dans le conduit 6, comme montre la figure 4, car dans ce cas, cet élément 8, ne peut pas reculer à travers l'orifice 21 du capuchon 20, pour appuyer ce dernier contre la portion grossie 16 de cette tige de manoeuvre et qui est empêché par la configuration échelonnée de l'élément 8.

De cette façon, à chaque couple de déplacements en arrière et en avant ultérieur, consécutifs, de la tige de manoeuvre 5, correspond l'avancement d'un pas ou échelon, de l'élément auxiliaire 8, qui avance à travers le piston 4.

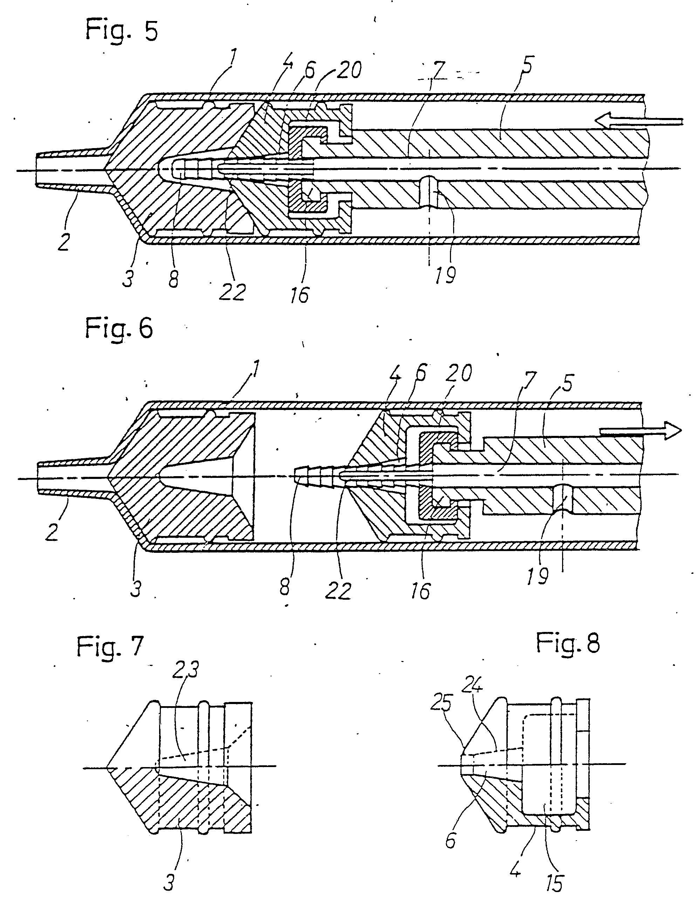

Lorsque dans cet avancement, pas à pas, de l'élément auxiliaire 8, le sillon générateur 22, arrive à l'extrémité du conduit 6, du piston, selon montre la figure 5, ce sillon 22,permet la communication avec l'atmosphère de l'enceinte entre les pistons 3, 4, (par son passage et par les conduits 7 et 19), avec quoi, comme montre la figure 6, on ne pourra actionner alors qu'en arrière le piston libre 3, la fonction d'aspiration de la seringue devenant impossible.

Dans les figures 7 et 8, on trouve la répresentation graphique des pistons 3 et 4, le premier disposant d'une cavité 23, pour permettre le déplacement vers celle-ci de l'extrémité d'un élément auxiliaire 8, et présentant le deuxième une vaste cavité 15 et un conduit 6, qui fait communiquer cette cavité 15 avec l'extrémité du piston 4, comprenant une portion tronconique (24) parachevée par un secteur cylindrique 25, ce dernier afin d'établir un ajustement hermétique sur la section de l'élément tubulaire 8, et en fournissant une friction modérée sur cet élément 8.

Le fonctionnement de la seringue décrite serait possible en employant un seul piston, quoique dans ce cas, lorsque l'élément auxiliaire 8 arrive à mettre en communication l'enceinte avant de ce piston avec l'atmosphère, la seringue devient inopérante et si on essaie de pousser le piston le liquide qui se trouve devant lui passera de l'autre côté de ce piston, l'injection effective n'étant pas possible.

Il faut considérer également dans l'essentialité de cette invention, une disposition des éléments cités, où l'élément auxiliaire au lieu d'avancer vers l'extrémité de la seringue où l'aiguille s'accouple, avance, pas à pas, comme il a été expliqué, mais en sens inverse.Dans ce dernier cas, si la seringue ne comportait qu'un piston, l'élément auxiliaire 8, tant qu'il serait visible, lorsqu'il dépasserait par dessus ledit piston, fournirait une indication bien visible à l'usager en ce sens que la seringue est encore opératrice, tandis que lorsqu'il disparaît à l'intérieur du piston, il se rendra clairement compte que les phases que fournit cette seringue sont en train de finir.

Il faut considérer également accessoire la forme de l'élément auxiliaire 8, ainsi que le passage de prise atmosphérique 19, car en considérant que ledit élément 8, puisse avancer dans une direction opposée à celle expliquée, il pourrait opérer comme un simple bouchon, la communication atmosphérique de la region avant du piston 4, étant établie, une fois le bout de cet élément 8, a sorti de la partie cylindrique 25 du conduit 6, par ce même conduit 6, et par un passage établi à travers de la paroi bassale annulaire de la cavité 15, à l'intérieur du piston 4, par exemple.

L'élément auxiliaire 8, cité, se placera à la position initiale de montage de la seringue, à la position exacte reliée au piston 4 et tige 5, en l'insérant à travers le conduit 7, ou à travers le conduit 6 selon son sens d'avancement et avec l'aide d'un jauge-poussoir.

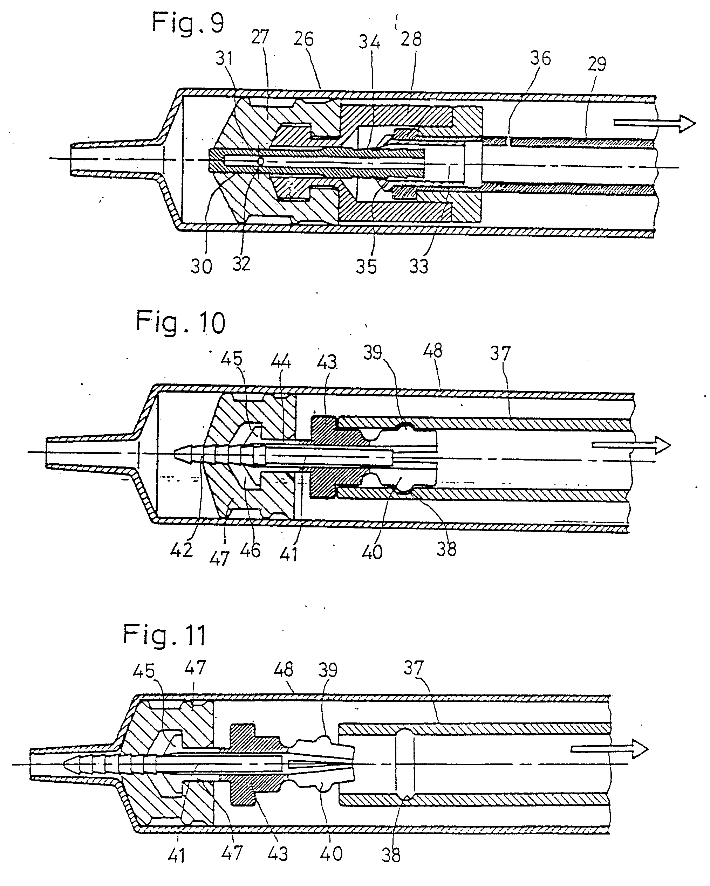

La figure 9 montre une seringue dont le corps 26, loge un seul piston 27, couplé en montage coulissant à son intérieur, relié à une pièce 28, qui a accouplé à son intérieur l'extrémité grossie d'une tige 29, de manoeuvre.Associé par ces extrémités à la tige 29, et piston 27, on observe un élément auxiliaire 30, constitué par un tuyau fermé par une de ses extrémités dont la partie est logée avec raccordement de friction dans un conduit 31 du piston 27 présentant en sa zone éloignée de son extrémitée fermée, un ou plusieurs orifices, 32, qui restent bouchés pendant une partie du déplacement longitudinal dudit élément auxiliaire 30. L'autre extrémité de cet élément 30, reste logée avec raccordement de friction/engrenage, en un conduit 33 pratiqué à la portion de l'extrémité de la tige 29 et présente une configuration extérieure échelonnée 34, au moyen de laquelle et à l'aide d'un moyen de pince sous forme des pattes 35, qui l'encerclent, et établies à l'extrémité du conduit 33, on obtient le raccordement cité d'engrenage/friction.Le conduit 33 présente une ouverture 36,de prise atmosphérique.

Dans les figures 10 et 11, une réalisation de la seringue est dessinée, ou une tige de manoeuvre 37, sous forme de tuyau présente à la paroi interne d'une zone immédiate à un de ses embouts un sillon annulaire 38, où reste couplé un profil en anneau 39, en saillie d'une pièce 40, insérée dans ledit tube 37, et étendue par l'introduction travers lui de la section d'un élément auxiliaire 41, formé par un axe lisse ayant une portion distale 42, à paroi échelonnée que traverse un piston 47.Ladite pièce extensible 40, est continuée par une portion doublement cylindrique 43, coaxiale du tuyau 37, qui se prolonge à son tour par un goulot tubulaire 44, fini par des pattes 45.Les parties 44, et 45, restent retenues au sein d'un creux 46, d'un piston 47, installé a l'intérieur du corps 48,de la seringue en permettant le déplacement en double sens de ladite portion de l'extrémité 44, 45,de la tige de manoeuvre.

Dans cette réalisation, comme il a été expliqué, après un certain nombre de déplacements de la tige de manoeuvre 37, l'élément auxiliaire 41, abandonne l'intérieur de la pièce 40, qui ne recevant pas un effort d'expansion appropié, libère la tige de manoevre 37, de son association avec le piston 47, d'après ce qui est illustré à la figure 11.

Les figures 12, 13 et 14 montrent des variantes équivalant a la seringue des figures 1 à 6, où uniquement la forme de l'élément auxiliaire a été modifiée coté à ces figures par les chiffres 49-50 et 51, ainsi que les moyens de retenue dudit élément à l'extrémité de la tige de manoeuvre.Aux effets de simplification, on a conservé les numéros aux pièces communes.

Dans la figure 12, l'élément auxiliaire 49, est formé par un tuyau lisse ayant un orifice 52, à son extrémité qui reste inséré dans le conduit 6, du piston 4, et son autre pointe reste disposée à l'intérieur d'un logement 53, de l'extrémité de la tige 5, qui présente une sortie de prise atmosphérique 22.

La figure 13, est equivalente à la précédente mais l'extrémité 54, de l'élément auxiliaire 50, reste retenu à la portion terminale de la tige 5, pour un pivot 55, chargé par une rondelle élastique 56.

Finalement, la figure 14 introduit la variante de retenue de l'élément auxiliaire 51, à configuration tubulaire lisse à l'extrémité de la tige 5, au moyen d'un joint torique 57.

Il faut remarquer après cette description, que l'essentialité de cette invention se trouve en un particulier raccordement de la tige de manoeuvre au piston, qui fournit un déplacement relatif de la première par rapport au second, avant la course de ce dernier au long du corps de la seringue, en correspondance avec toute action de recul ou avancement du piston de manoeuvre, lequel déplacement est transféré à un élément auxiliaire, relié par ses deux boutes au piston et à la tige de manoeuvre, avec un raccordement de conditions d'actuation differentielles.On a proposé par sa simplicité et effectivité, que le déplacement dudit élément auxiliaire puisse arriver à établir une prise atmosphérique de la region avant du piston, restant alors la seringue inutile à aspirer, mais il pourrait produire d'autres effets avec un résultat tout à fait équivalent.

Il faut considérer également accesoires les moyens employés pour l'immobilisation de la tige de manoeuvre par rapport au piston, avant l'utilisation de la seringue, d'autres solutions déjà en pratique dans ce domain technique étant possible.

Les moyens employés pour empêcher l'extraction du piston et/ou tige du corps de la seringue peuvent être différents de ceux qu'on a expliqué qui ne doivent se prendre en un sens limitatif.

Pour une plus grande sécurité on peut employer , pour constituer le corps de la seringue, un matérial éclatable face aux efforts de frappement ou sciement.

Une fois suffisamment décrite cette invention, pour que son essentialité puisse être comprise par un homme du métier, il est demandé d'étendre son objet aux variations de détail n'en altérant pas l'essentialité, telles que différentes configurations de la tige 5, pistons 3, 4, élément auxiliaire 8, élément élastique 20, moyens d'immobilisation 13 et 14, et de retenue 9, qui remplissent une fonction équivalente à celle décrite, ses caractéristiques basiques étant resumées dans les revendications suivantes.

**Revendications**

1. Seringue pour l'injection de fluide à mécanisme appliqué à son inutilisation programmée formée par un corps tubulaire allongé creux, terminé en une de ses extrémités par un conduit adapté pour recevoir branchée une aiguille creuse ayant une extrémité en biais et, disposé à son intérieur, en montage coulissant, un piston hermétiquement ajusté, relié à une tige de manoeuvre, caracterisée en ce qu'une portion de l'extrémité de ladite tige de manoeuvre reste mutuellement reliée au piston ou à un corps solidaire de celui-ci, par un raccordement qui fournit un déplacement relatif bidirectionnel, à longueur délimitée, de ladite portion de l'extrémité de la tige par rapport au piston ou corps qui lui est solidaire, en correspondance avec toute action de recul et/ou avancement du piston de manoeuvre, en intégrant de plus un élément auxiliaire monté avec possibilités de coulissement à un seul sens, le long du corps de la seringue, associé par ses deux extrémités à des raccordements spécifique de friction ou engrenage au piston et à l' extrémité de la tige de manoeuvre, possédant à cet éffet des moyens d'actuation différencié, de retenue et de passage à travers ledit élément auxiliaire, qui adopte une configuration contribuant à l'unidirectionnalité et longueur de ses déplacements, en constituant l'ensemble tige, piston et élément auxiliaire, un mécanisme au moyen duquel il est possible d'agir sur divers activateurs pour inutiliser la seringue, dont le corps a une configuration et enclechement qui empêchent le mouvement relatif entre tige de manoeuvre et piston jusqu'au moment de l'utilisation de la seringue et une configuration ou élément de butée qui empêche l'extraction du piston et/ou tige du corps de la seringue.

2. Seringue, selon la revendication précédente, caracterisée en ce comporte :

a) une cavité, définie à l'intérieur dudit piston ou au sein d'un corps qui y est uni, apte pour y loger et retenir une portion grossie, tout à fait au bout de ladite tige de manoeuvre, l'envergure de cette cavité étant telle qu'elle per met un déplacement relatif, dans le sens de l'axe de symetrie du corps tubulaire, à deux sens, de la portion grossie de la tige, par rapport au piston ou au corps qui y est relié.

b) des moyens tels qu'une configuration delimitant avec précision la longueur du déplacement relatif à double sens de la portion finale grossie, de la tige de manoeuvre, par rapport au piston.

c) un conduit faisant communiquer la cavité du piston citée avec le côté avant de ce piston face à face avec le bout porteur de l'aiguille de la seringue.

d) un conduit ou logement axial à la tige de manoeuvre.

e) un passage de prise atmosphérique de la cavité du piston ou du logement de la tige de manoeuvre.

f) un élément en matériau flexible tel qu'un capuchon, capuche ou feuille, accouplé, couvrant la portion du bout grossi de la tige de manoeuvre, avec un orifice correspondant en alignement avec ledit conduit ou logement axial de la tige de manoeuvre.

g) un élément auxiliaire, formé par un axe ou tenon tubulaire présentant avantageusement une configuration échelonnée au moins d'une partie de sa surface externe, avec un sillon générateur couvrant une partie de son corps, dont l'élément auxiliaire reste placé avec une extrémité logée dans le conduit ou logement axial de la tige de manoeuvre inserée à travers l'orifice de l'élément élastique qui ferme ce conduit/logement et avec son autre extrémité introduite et hermétiquement ajustée dans le conduit dudit piston.

h) une configuration ou élément de butée qui empêche l'extraction du piston et /ou tige de manoeuvre, une fois ils sont installés dans le corp de la seringue.

i) des moyens pour empêcher le mouvement relatif entre la tige de manoeuvre et le piston jusqu'au moment de l'utilisation de cette seringue.

3. Seringue, selon la revendication 2, caracterisée en ce que la configuration échelonnée de l'élément auxi liaire consiste en une pluralité de secteurs tronconiques égaux, coaxiales, raccordés en continuité, leur base plus petite correspondant à la zone avant de l'élément, dans le sens de son avancement dans la seringue.

4. Seringue selon la revendication 2, caracterisée en ce que l'hauteur de chaque secteur, tronconique ou échelon de l'élément auxiliaire tubulaire, correspond à la longueur du déplacement relatif entre le bout de la tige et du piston, permi à chaque phase d'avancement et de recul du premier.

5. Seringue selon la revendication 2, caracterisée en ce qu'il comporte un deuxième piston libre, montée hermétiquement ajustée, en montage glissant et placée en avant de l'overture de sortie de la seringue, dont ce piston libre reste adjacent à l'autre piston, lié à la tige de manoeuvre, jusqu'au moment où la seringue devient inutilisée.

6. Seringue selon les revendications 2 et 5, caracterisée en ce que ledit piston libre a une cavité axiale.

7. Seringue selon la revendication 2, caractérisée en ce que les moyens pour délimiter la longueur du déplacement relatif entre tige de manoeuvre et piston, sont formés par la cavité interne de ce piston et par un tronçon avec réduction de section de cette tige qui unit sa portion grossie, du bout, avec le reste du corps, plus gros.

8. Seringue, selon la revendication 2, caractérisée en ce que l'embout du corps tubulaire de la seringue, présente un étranglement, qui empêche l'extraction du piston et/ou de la tige de manoeuvre.

9. Seringue, selon la revendication 2, caractérisée en ce que la tige de manoeuvre et le piston présentent des configurations mâle-femelle, dans la region de l'embout du corps tubulaire qui restent couplés dans une position particulière, constituant lesdits moyens d'immobilisation du mouvement relatif entre la tige de manoeuvre et piston.

10. Seringue, selon la revendication 1, caracterisée en ce que dans sa region d'embout présente une configuration de butée du déplacement de la tige de manoeuvre.

11. Seringue selon la revendication 1, caractérisée en ce que l'élément auxiliare est formé par un tuyau fermé par une de ses extrémités, présentant à une certaine distance de ce bout quelques trous, qui reste logé dans un conduit qui traverse le piston, ayant une partie de sa surface extérieure échelonnée, raccordée à la tige de manoeuvre.

12- Seringue, selon la revendication 1, caractérisée en ce que l'élément auxiliaire mobile, tubulaire, est completement lisse.

13. Seringue, selon la revendication 1, caractérisée en ce que dans la region inmediate à la partie extreme grossie de la tige de manoeuvre il y a des configurations en pince elastique sous forme de pattes qui encerclent l'extrémité de la section de l'élément auxiliare inséré dans cette partie.

14. Seringue, selon, la revendication 1, caracterisée en ce que dans l'embout du conduit/logement dans l'extrémité de la tige de manoeuvre ou se loge une des extrémités de l'element auxiliare, un moyen de jointe agît en retenue.

15. Seringue, selon le revendication 1, caractérisée en ce que l'élément auxiliaire mobile, présente une pluralité d'appendices elastiques en saillie et le conduit de son logement à l'extrémité de la tige de manoeuvre, a ses parois lisses.

16. Seringue, selon la revendication 1, caractérisée en ce qu'elle intégre un piston unique, étant constitué la region extrême de la tige de manoeuvre relié à ce piston, par deux parties couplées opérativement, une d'elles formé par la dite tige de manoeuvre, sous forme de tuyau, et qui présente à la paroi interne d'une zone inmediate à un de ses embouts, un sillon annulaire, où reste couplé un profil en anneau, en saillie, d'une pièce insérée dans le dit tube et étendue par l'introduction à son travers de la section de l'élément auxiliaire mobile quand on actionne la seringue, laquelle pièce reste raccordée par son autre bout au piston, logé dans sa cavité.

EP 0 304 386 A2

Fig. 1

Fig. 2

Fig. 3

Fig. 4

Fig. 5

Fig. 6

Fig. 7

Fig. 8

Fig. 9

Fig. 10

Fig. 11

Fig. 12

Fig.13

Fig.14